# EUROPEAN PATENT APPLICATION

(11) **EP 2 898 816 A1**
(43) Date of publication of application: **29.07.2015**
(21) Application number: 13838319.5
(22) Date of filing: 22.08.2013
(51) Int. Cl.: A61B 1/00

(54) **INSERTION AID, INSERTION BODY, AND INSERTION DEVICE**

(30) Priority: 19.09.2012 JP 2012206170
(71) Applicant: Olympus Medical Systems Corp., Tokyo 151-0072 (JP)
(72) Inventor: NISHIIE, Takehiro, Hachioji-shi Tokyo 192-8512 (JP); NAITO, Kimihiko, Hachioji-shi Tokyo 192-8512 (JP)
(74) Representative: Schicker, Silvia
(86) International application number: PCT/JP2013/072479
(87) International publication number: WO 2014/045795

(57) **Abstract**

A cylindrical rotation unit provided outside an elongated insertion body which defines a central axis being substantially parallel with a longitudinal axis of a channel having an opening end portion in which a rotation gear having external teeth of a rotation force transmission unit is detachably provided and which is open to the outside, includes an internal gear which includes internal teeth configured to mesh with the external teeth of the rotation gear and which is rotatable around the central axis while the rotation gear is disposed in the opening end portion of the channel, and a tube which is configured to support the internal gear on an inner circumferential surface of the tube.

## Description

### Technical Field

This invention relates to an insertion apparatus to be inserted into a lumen, an insertion body, and an insertion assist device which assists the insertion into the lumen.

### Background Art

For example, an insertion portion of an endoscope according to Jpn. Pat. Appln. KOKAI Publication No. 2007-185394 has therein a rotation force transmission unit which includes a rotation gear, and a drive shaft fixed to the proximal end of the rotation gear. If rotation force is transmitted to the rotation gear through the drive shaft of the rotation force transmission unit, a cylindrical spiral portion (insertion assist device) having internal teeth in mesh with the rotation gear rotates together with the rotation of the rotation gear. As a result of the rotation of the spiral portion, the insertion portion is inserted into a lumen from a near side to a far side, or removed from the far side to the near side.

The endoscope is often washed, disinfected, and sterilized, and then reused. Therefore, there has been a request that the insertion assist device which assists the insertion into the lumen be easily attached to and detached from an insertion body of the insertion portion and that the washing performance of the insertion body from which the insertion assist device is detached be improved.

### Summary of Invention

It is an object of the present invention to provide an insertion assist device which is easily attached to and detached from an insertion body of the insertion portion so as to improve detergent properties of the insertion body, an insertion body which can improve the detergent properties when the insertion assist device is detached therefrom, and an insertion apparatus including the insertion assist device and the insertion body.

According to one embodiment of the present invention, a cylindrical insertion assist device in an insertion apparatus, the insertion assist device provided outside an elongated insertion body which defines a central axis being substantially parallel with a longitudinal axis of a channel having an opening end portion in which a rotation gear having external teeth of a rotation force transmission unit is detachably provided and which is open to the outside, includes an internal gear which includes internal teeth configured to mesh with the external teeth of the rotation gear and which is rotatable around the central axis while the rotation gear is disposed in the opening end portion of the channel; and a tube which is configured to support the internal gear on an inner circumferential surface of the tube.

### Brief Description of Drawings

FIG. 1 is a schematic diagram showing an endoscope system according to first and second embodiments of the present invention;
FIG. 2A is a schematic perspective view showing a rotation force transmission unit which is attachable to and detachable from an endoscope of the endoscope system according to the first embodiment;
FIG. 2B is a schematic front view showing the rotation force transmission unit in FIG. 2A viewed from the direction of an arrow 2B;
FIG. 3 is a schematic diagram showing an operation portion of the endoscope of the endoscope system according to the first embodiment when viewed from the rear side in FIG. 1;
FIG. 4 is a schematic longitudinal sectional view showing part of an active bending portion, a passive bending portion, and part of a first flexible tube in an insertion portion of the endoscope of the endoscope system according to the first embodiment;
FIG. 5 is a schematic longitudinal sectional view showing a flexible tube connection portion between the first flexible tube and a second flexible tube in the insertion portion of the endoscope of the endoscope system according to the first embodiment;
FIG. 6 is a schematic longitudinal sectional view showing how the rotation force transmission unit is attached to a channel of the flexible tube connection portion between the first flexible tube and the second flexible tube in the insertion portion of the endoscope of the endoscope system according to the first embodiment;
FIG. 7 is a schematic longitudinal sectional view showing how the rotation force transmission unit is attached to the channel of the flexible tube connection portion between the first flexible tube and the second flexible tube, and a rotation unit is attached to the outside of the flexible tube connection portion, in the insertion portion of the endoscope of the endoscope system according to the first embodiment;
FIG. 8 is a schematic cross sectional view taken along the line VIII-VIII in FIG. 7;
FIG. 9A is a schematic longitudinal sectional view showing how a rotation shaft of a driving source is to be connected to the proximal end of a drive shaft projecting toward the proximal side relative to an exit where the rotation force transmission unit is inserted through an insertion body, in the insertion portion of the endoscope of the endoscope system according to the first embodiment;
FIG. 9B is a schematic cross sectional view taken along the line 9B-9B in FIG. 9A;
FIG. 10A is a schematic longitudinal sectional view showing how the rotation force transmission unit is attached to the channel of the flexible tube connection portion between the first flexible tube and the second flexible tube, and the rotation unit is attached to the outside of the flexible tube connection portion, in the insertion portion of the endoscope of the endoscope system according to the second embodiment; and
FIG. 10B is a schematic cross sectional view of holding pieces of the rotation unit taken along the line 10B-10B in FIG. 10A.

### Brief Description of Embodiments

Embodiments of this invention will be described with reference to the drawings.

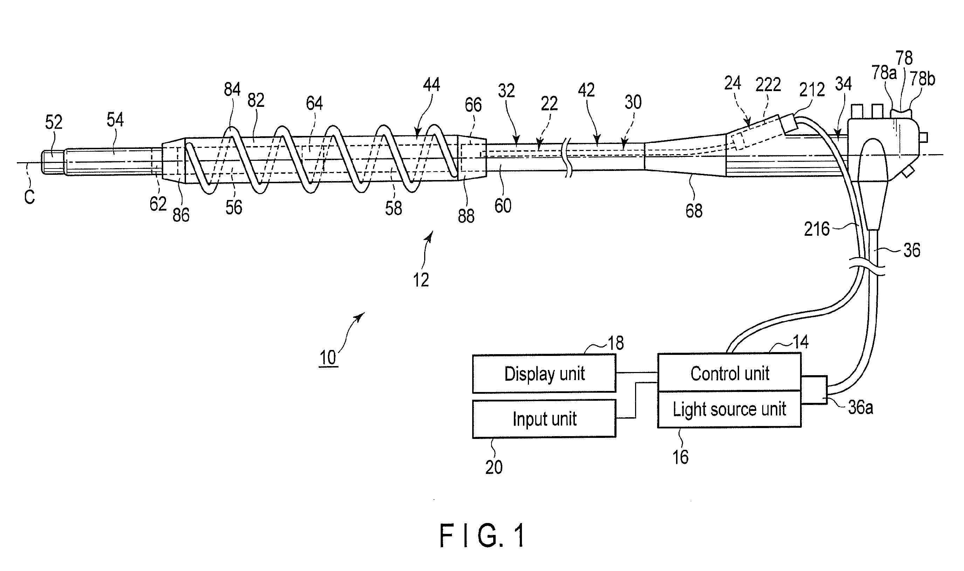

The first embodiment is described with reference to FIG. 1 to FIG. 9B.

As shown in FIG. 1, an endoscope system 10 according to this embodiment includes an endoscope (insertion apparatus) 12, a control unit 14 which includes an unshown image processing unit such as an image processor and which controls the whole endoscope system 10, a light source unit 16, a display unit 18 such as a monitor, and an input unit 20 such as a keyboard and a mouse.

The light source unit 16 is electrically connected to the control unit 14. The display unit 18 and the input unit 20 are electrically connected to the control unit 14. When a light source is provided in a distal hard portion 52 of a later-described insertion portion 32 of the endoscope 12, the light source unit 16 can be unnecessary.

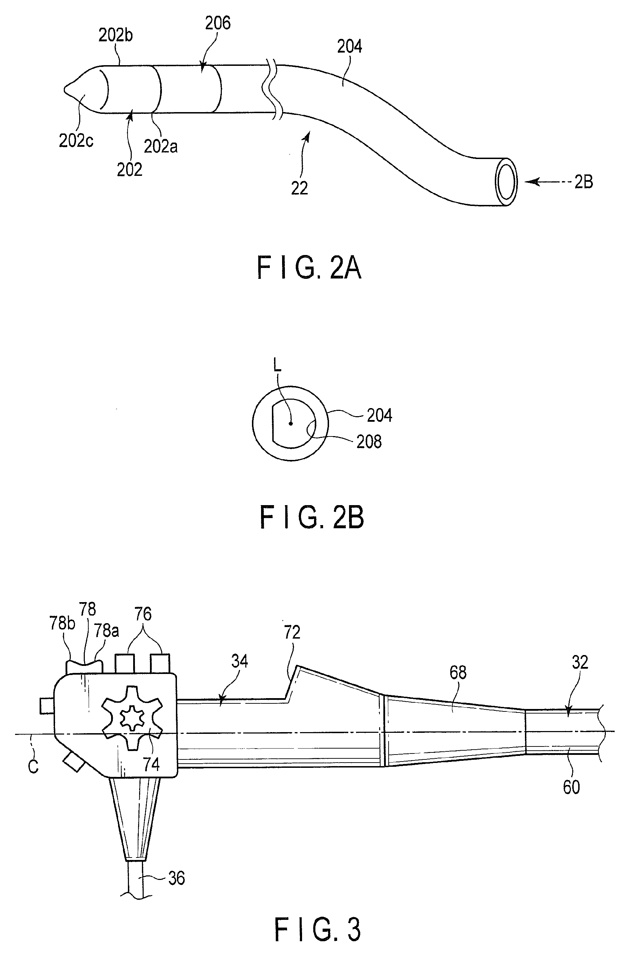

In the endoscope system 10 according to this embodiment, a rotation force transmission unit (insertion body) 22 shown in FIG. 2A and a driving source 24 shown in FIG. 1 can be attached to and detached from the endoscope 12 from the side of a later-described operation portion 34. That is, the endoscope 12 has a channel 30 which defines an insertion pathway to attach and detach the rotation force transmission unit 22 and the driving source 24. The channel 30 then defines a longitudinal axis L on which the rotation force transmission unit 22 is inserted at a position which is out of alignment with a central axis C of a later-described insertion body 42. The longitudinal axis L of the channel 30 and the central axis C of the insertion body 42 are parallel to each other in a gear location cavity 142 in particular.

The endoscope 12 includes the insertion portion 32 to be inserted into a narrow hole such as a lumen, and the operation portion 34 provided at the proximal end of the insertion portion 32. The insertion portion 32 is inserted into a lumen such as the inside of a large intestine or the inside of a small intestine. One end of a universal cable 36 is connected to the operation portion 34. A scope connector 36a is provided at the other end of the universal cable 36. The scope connector 36a is connected to the control unit 14 and the light source unit 16.

The insertion portion 32 includes the elongated insertion body 42, and a rotation unit (insertion assist device) 44 which is detachably attached to the outer circumference of the insertion body 42 and which is rotatable around the later-described central axis C of the insertion body 42.

The insertion body 42 includes, in order from the distal end to the proximal end, the distal hard portion 52, an active bending portion 54, a passive bending portion 56 which passively bends in response to the application of external force, a first flexible tube 58, and a second flexible tube 60. The central axis C of the insertion portion 32 is defined by the distal end of the insertion body 42 (the distal end of the distal hard portion 52) and the proximal end thereof (the proximal end of the second flexible tube 60).

The active bending portion 54 and the passive bending portion 56 are connected to each other by a bent tube connection portion 62. The passive bending portion 56 and the first flexible tube 58 are connected to each other by an intermediary connection portion 64. The first flexible tube 58 and the second flexible tube 60 are connected to each other by a flexible tube connection portion 66. A break prevention 68 is provided between the second flexible tube 60 and the operation portion 34. That is, the insertion body 42 has the bent tube connection portion 62, the intermediary connection portion 64, and the flexible tube connection portion 66, in addition to the distal hard portion 52, the active bending portion 54, the passive bending portion 56, the first flexible tube 58, and the second flexible tube 60.

The distal ends of various extensive components 12a extended inside the endoscope 12 such as an observation optical system, an illumination optical system, and a treatment instrument channel are fixed to the distal hard portion 52 shown in FIG. 1. The extensive components 12a of the observation optical system and the illumination optical system are respectively connected to the connector 36a through the insertion body 42, the operation portion 34, and the universal cable 36. The extensive component 12a, that is, a channel tube of the treatment instrument channel is connected to the operation portion 34 through the insertion body 42.

FIG. 3 shows the opposite side surface of the operation portion 34 shown in FIG. 1. As shown in FIG. 3, the operation portion 34 has a treatment instrument insertion opening 72 to which the proximal end of the extensive component 12a of the treatment instrument channel is connected. Therefore, a treatment instrument inserted from the treatment instrument insertion opening 72 projects from the distal end of the distal hard portion 52 through the extensive component 12a, that is, the treatment instrument channel.

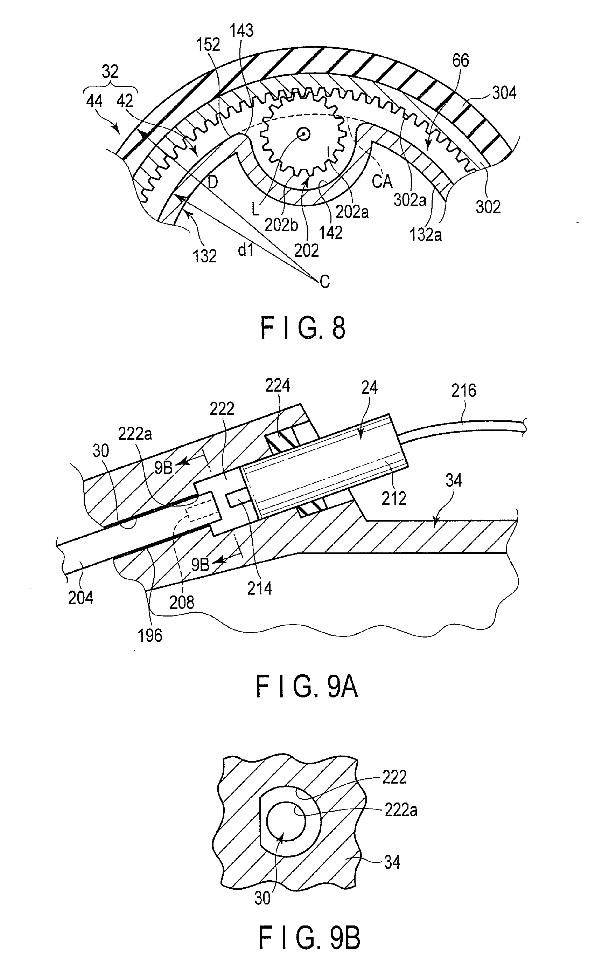

Although described later, the operation portion 34 includes an attachment portion 222 (see FIG. 9A) which is provided parallel with the treatment instrument insertion opening 72 and which the driving source 24 can be attached to and detached from.

As shown in FIG. 3, a bending operation knob 74 serving as a bending operation input portion to which a bending operation of the active bending portion 54 is input is provided on the outer surface of the operation portion 34. Inside the operation portion 34, the proximal end of an unshown bending wire for curving the active bending portion 54 is connected to the bending operation knob 74. The bending wire extends inside the insertion body 42 (insertion portion 32) along the central axis C, and has its distal end connected to the distal end of the active bending portion 54. In response to the bending operation in the bending operation knob 74, the active bending portion 54 is curved by the pulling of the bending wire. The passive bending portion 56 is passively curved by direct application of external force or by indirect application of external force via the active bending portion 54. For example, if external force in a direction perpendicular to the central axis C is applied to the passive bending portion 56, the passive bending portion 56 is bent. If external force in a direction perpendicular to the central axis C is applied to the curved active bending portion 54, the external force is also applied to the passive bending portion 56 via the active bending portion 54, and the passive bending portion 56 is curved.

The operation portion 34 is provided with various switches 76 such as an air/water supply switch and a suction switch. The operation portion 34 is also provided with a rotational operation input switch 78 which outputs, to the control unit 14, a signal to relatively rotate the rotation unit 44 around the central axis C of the insertion body 42. For example, if a position indicated by the sign 78a is pressed to incline the rotational operation input switch 78, the rotational operation input switch 78 outputs, to the control unit 14, a signal to rotate the rotation unit 44 in a first direction. If a position indicated by the sign 78b is pressed to incline the rotational operation input switch 78, the rotational operation input switch 78 outputs, to the control unit 14, a signal to rotate the rotation unit 44 in a second direction opposite to the first direction.

The rotation unit 44 on the outer circumference of the insertion body 42 includes a tube body 82 extending along the central axis C, a fin portion 84 extending spirally relative to the central axis C on the outer circumference of the tube body 82, a tubular tube distal portion 86 provided at the distal end of the tube body 82, and a tube proximal portion 88 provided at the proximal end of the tube body 82.

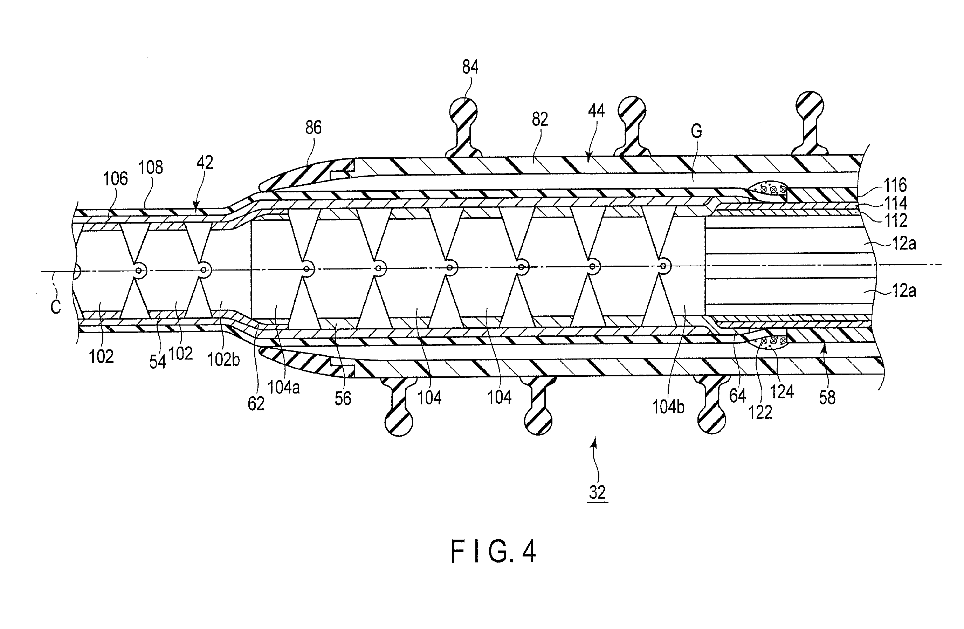

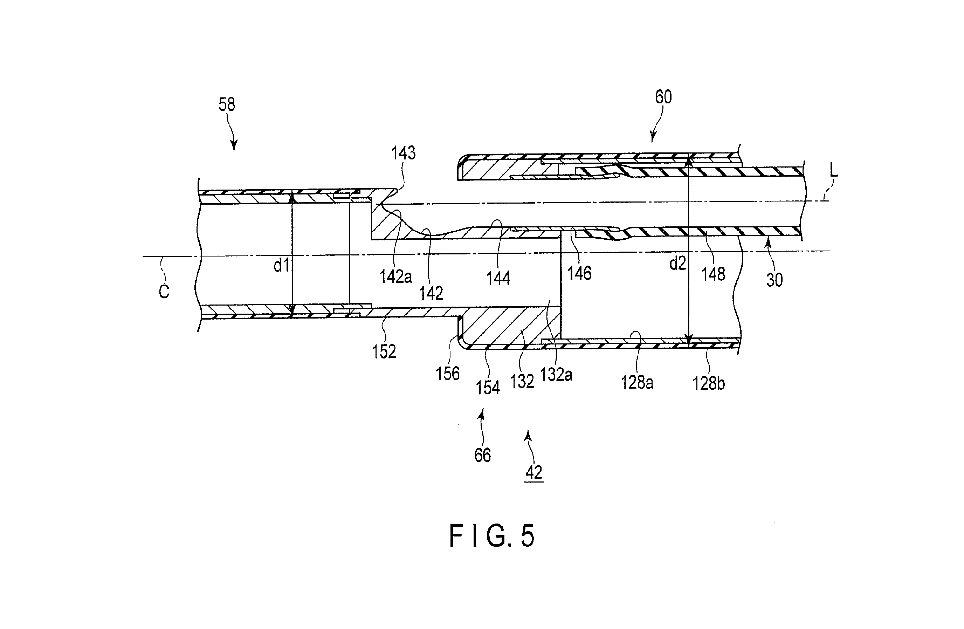

The configurations of the insertion body 42 and the rotation unit 44 in the vicinity of the passive bending portion 56 are shown in FIG. 4. The configurations of the insertion body 42 and the rotation unit 44 in the vicinity of the flexible tube connection portion 66 are shown in FIG. 5 to FIG. 8.

As shown in FIG. 4, the active bending portion 54 includes metallic first joint rings 102. Each of the first joint rings 102 is pivotally coupled to the adjacent first joint ring 102. The distal end of the above-mentioned unshown bending wire is fixed to an unshown first joint ring located on the most distal direction side. When the bending wire is pulled, the first joint ring 102 pivots relative to the adjacent first joint ring 102, and the active bending portion 54 is bent.

The passive bending portion 56 includes metallic second joint rings 104. Each of the second joint rings 104 is pivotally coupled to the adjacent second joint ring 104. No wire guide which supports the bending wire is provided in each of the second joint rings 104. Therefore, in response to external force applied in the direction perpendicular to the central axis C, the second joint ring 104 pivots relative to the adjacent second joint ring 104, and the passive bending portion 56 is bent.

A first joint ring 102b located on the most proximal direction side is fixedly fitted to a second joint ring 104a located on the most distal direction side. The first joint ring 102b is fixed to the second joint ring 104a so that the bent tube connection portion 62 is formed between the active bending portion 54 and the passive bending portion 56. In the bent tube connection portion 62, the first joint ring 102b is fixed to the second joint ring 104a, and the thickness of a metallic portion formed by the first joint ring 102b and the second joint ring 104a is larger. Therefore, the bent tube connection portion 62 is less flexible than the active bending portion 54 and the passive bending portion 56, and is therefore not bent by the external force applied in the direction perpendicular to the central axis C.

The outer circumferences of the first joint rings 102 and the second joint rings 104 are covered with a metallic braided tube (braid) 106. The outer circumference of the braided tube 106 is covered with an outer tube 108. The outer tube 108 of the bending portion is made of, for example, fluoro rubber.

As shown in FIG. 4, the first flexible tube 58 is provided with a metallic first spiral tube (first flex) 112. The outer circumference of the first spiral tube 112 is covered with a metallic first braided tube (first braid) 114. The outer circumference of the first braided tube 114 is covered with a first outer tube 116. The first outer tube 116 is made of a material lower in flexibility than the outer tube 108 of the active bending portion 54 and the passive bending portion 56, such as a mixed resin material of polyurethane and polyester. When external force is applied, the first spiral tube 112 becomes lower in curving performance than a coupler of the first joint rings 102 and a coupler of the second joint rings 104. Therefore, the first flexible tube 58 becomes lower in flexibility than the active bending portion 54 and the passive bending portion 56. However, the first flexible tube 58 is provided to have such a degree of flexibility that the first flexible tube 58 is curved by the external force applied in the direction perpendicular to the central axis C.

A second joint ring 104b located on the most proximal direction side is fixedly fitted to the first spiral tube 112 and the first braided tube 114. The second joint ring 104b is fixed to the first spiral tube 112 and the first braided tube 114 so that the intermediary connection portion 64 is formed between the passive bending portion 56 and the first flexible tube 58. In the intermediary connection portion 64, the second joint ring 104b is fixed to the first spiral tube 112 and the first braided tube 114, and the thickness of a metallic portion formed by the second joint ring 104b, the first spiral tube 112, and the first braided tube 114 is larger. Therefore, the intermediary connection portion 64 is less flexible than the passive bending portion 56 and the first flexible tube 58, and is therefore not bent by the external force applied in the direction perpendicular to the central axis C.

The proximal end of the outer tube 108 of the bending portion and the distal end of the first outer tube 116 are located in the intermediary connection portion 64. Between the outer tube 108 of the bending portion and the first outer tube 116, the first outer tube 116 and the outer tube 108 of the bending portion are wound with a thread 122, and are covered with an adhesive agent 124.

Although partly not shown, the extensive components 12a are inserted through the spaces inside the first joint rings 102, the bent tube connection portion 62, the second joint rings 104, the intermediary connection portion 64, and the first spiral tube 112.

The second flexible tube 60 shown in FIG. 5 to FIG. 7 is similar in configuration to the first flexible tube 58. Therefore, although not described in detail, the second flexible tube 60 is provided to have such a degree of flexibility that the second flexible tube 60 is curved by the external force applied in the direction perpendicular to the central axis C. A tubular member indicated by the sign 128a in FIG. 5 to FIG. 7 represents a spiral tube and a braided tube provided on the outer circumference of the spiral tube. A circular member indicated by the sign 128b is used as an outer tube. Therefore, the first flexible tube 58 and the second flexible tube 60 have the same configuration.

It is preferable that the outer tube 128b of the second flexible tube 60 not only covers the outside of the spiral tube indicated by the sign 128a and the outside of the braided tube provided on the outer circumference of the spiral tube but also covers the outer circumferences of a large-diameter portion 154 and a step portion 156 of a later-described connection mouth ring 132.

As shown in FIG. 5 to FIG. 8, the flexible tube connection portion 66 between the first flexible tube 58 and the second flexible tube 60 includes the cylindrical connection cap (base) 132 made of, for example, a metallic material or a hard resin material. The connection mouth ring 132 has the central axis C defined by the insertion body 42.

The connection mouth ring 132 has a cavity 132a through which the extensive components 12a (see FIG. 4) that are not shown here are inserted.

The proximal end of the first flexible tube 58 is fixed to the distal end of the connection mouth ring 132. The distal end of the second flexible tube 60 is fixed to the proximal end of the connection mouth ring 132.

Although not shown, the extensive components 12a are inserted through the space inside the first flexible tube 58, the cavity 132a of the connection mouth ring 132, and the space inside the second flexible tube 60 that are in communication with one another.

Since the connection mouth ring 132 is made of, for example, a metallic material, the connection mouth ring 132 is formed to be unbendable relative to the first flexible tube 58 and the second flexible tube 60. For example, even if external force is applied to the flexible tube connection portion 66 by the inner wall of the large intestine, the flexible tube connection portion 66 does not bend.

As shown in FIG. 5 to FIG. 8, the gear location cavity 142 in which a rotation gear (rotation force transmission portion) 202 of the rotation force transmission unit 22 is located is formed in the connection mouth ring 132. This gear location cavity 142 includes an opening end portion 143 which is open to the outside of the connection mouth ring 132. As shown in FIG. 8, the cross section of the gear location cavity 142 at this position is, for example, substantially U-shaped. When the rotation gear 202 is provided in the gear location cavity 142, part (the upper part in FIG. 8) of the rotation gear 202 is located to project from the gear location cavity 142.

A circular arc CA indicated by a broken line in FIG. 8 represents a virtual outer circumferential surface of a later-described small-diameter portion 152 of the connection mouth ring 132 around the central axis C when the gear location cavity 142 is not formed. Although the circular arc CA is formed across a main body 202a of the rotation gear 202 in FIG. 8, the circular arc CA may be formed across a gear portion 202b if the gear portion 202b is allowed to mesh with later-described internal teeth 302a of the rotation unit 44. For example, it is only necessary that there be a root circle on the circular arc CA or on the outside of the circular arc CA and that there be a tip circle of the internal teeth 302a of the rotation unit 44 on the outside of the circular arc CA.

A support depression 142a which rotatably supports a later-described projection 202c at the distal end of the rotation gear 202 is provided in the gear location cavity 142.

A support portion 144 by which a later-described collar 206 of the rotation force transmission unit 22 is rotatably supported on the proximal side of the gear location cavity 142 is formed in the connection mouth ring 132. When the collar 206 of the rotation force transmission unit 22 is provided in the support portion 144, there is almost no gap between the outer circumferential surface of the collar 206 and the support portion 144, but the rotation around the longitudinal axis L, that is, the sliding around the longitudinal axis L is permitted.

A cylindrical channel mouth ring 146 for the rotation force transmission unit 22 is fixed to the connection mouth ring 132 at a position which is in communication with the gear location cavity 142 and the support portion 144. The channel mouth ring 146 is watertightly fixed to the connection mouth ring 132. The distal end of a channel tube 148 for the rotation force transmission unit 22 is fixed to the outer circumferential surface of the channel mouth ring 146. Therefore, the rotation force transmission unit 22 can be inserted through the channel tube 148. The channel tube 148 extends through the insertion body 42 in the proximal direction along the central axis C.

The channel tube 148 is watertightly fixed to the channel mouth ring 146, and is provided parallel with a treatment instrument channel tube which is one of the extensive components 12a.

The connection mouth ring 132 includes the small-diameter portion 152 on the distal side, the large-diameter portion 154 on the proximal side, and the step portion 156 located at the boundary between the small-diameter portion 152 and the large-diameter portion 154. As described above, the opening end portion 143 of the channel 30 which is in communication with the outside of the connection mouth ring 132 is formed in the gear location cavity 142 of the small-diameter portion 152. The small-diameter portion 152 and the large-diameter portion 154 are concentric with the central axis C. An outside diameter d2 of the large-diameter portion 154 is larger than an outside diameter d1 of the small-diameter portion 152. In other words, as shown in FIG. 5, the outside diameter d1 of the first flexible tube 58 coupled to the small-diameter portion 152 on the distal side of the connection mouth ring 132 is formed to be smaller than the outside diameter d2 of the second flexible tube 60 coupled to the large-diameter portion 154 on the proximal side of the connection mouth ring 132.

In FIG. 5 to FIG. 7 according to this embodiment, the first flexible tube 58 is directly coupled to the distal side of the small-diameter portion 152, and the second flexible tube 60 is directly coupled to the proximal side of the large-diameter portion 154. A mouth ring (not shown) may be located on the distal side of the small-diameter portion 152 so that the first flexible tube 58 is indirectly coupled to the small-diameter portion 152. A mouth ring (not shown) may also be located on the proximal side of the large-diameter portion 154 so that the second flexible tube 60 is indirectly coupled to the large-diameter portion 154.

Here, as shown in FIG. 2A, the rotation force transmission unit 22 includes the substantially columnar rotation gear (rotation force transmission portion) 202, a drive shaft 204, and the collar (rotating cylinder) 206 provided on the outer circumference of a connection portion between the rotation gear 202 and the drive shaft 204. The longitudinal axis L of the rotation force transmission unit 22 is defined by the rotation gear 202, the drive shaft 204, and the collar 206.

The rotation gear 202 includes the columnar main body 202a, the outer circumferential gear portion (external teeth) 202b formed on the outer circumference of the main body 202a, and the projection 202c formed at the distal end of the main body 202a. The outer circumferential gear portion 202b is rotatable around the longitudinal axis (rotation axis) L in the channel 30, and can mesh with the later-described internal teeth 302a of the rotation unit 44. The projection 202c formed at the distal end of the main body 202a of the rotation gear 202 is, for example, substantially conical. This projection 202c is rotatably supported by the support depression 142a provided in the gear location cavity 142.

There is almost no gap between the outer circumferential surface of the collar 206 provided on the proximal side of the rotation gear 202 and the support portion 144. The rotation gear 202 rotates while being doubly held by the support depression 142a and the support portion 144. Therefore, the rotation of the rotation gear 202 can be stabilized.

The drive shaft 204 extends toward the proximal side from the proximal end of the rotation gear 202 along the longitudinal axis L, and can rotate the rotation gear 202 if rotation force around the longitudinal axis L is applied to the proximal end of the drive shaft 204. The drive shaft 204 is formed into multiple layers by stacked metallic wires wound into a cylindrical net form, or formed by a multilayer wire in which right-handed and left-handed wire materials are stacked. The drive shaft 204 has a rotation following capability and flexibility.

As shown in FIG. 2B, the proximal end of the drive shaft 204 is, for example, circular. A D-shaped depression 208 into which a later-described D-shaped rotation shaft 214 of the driving source 24 is fitted is formed at the proximal end of the drive shaft 204. Therefore, the rotation of the rotation shaft 214 of the driving source 24 is transmitted to the drive shaft 204, and the rotation of the drive shaft 204 is transmitted to the rotation gear 202.

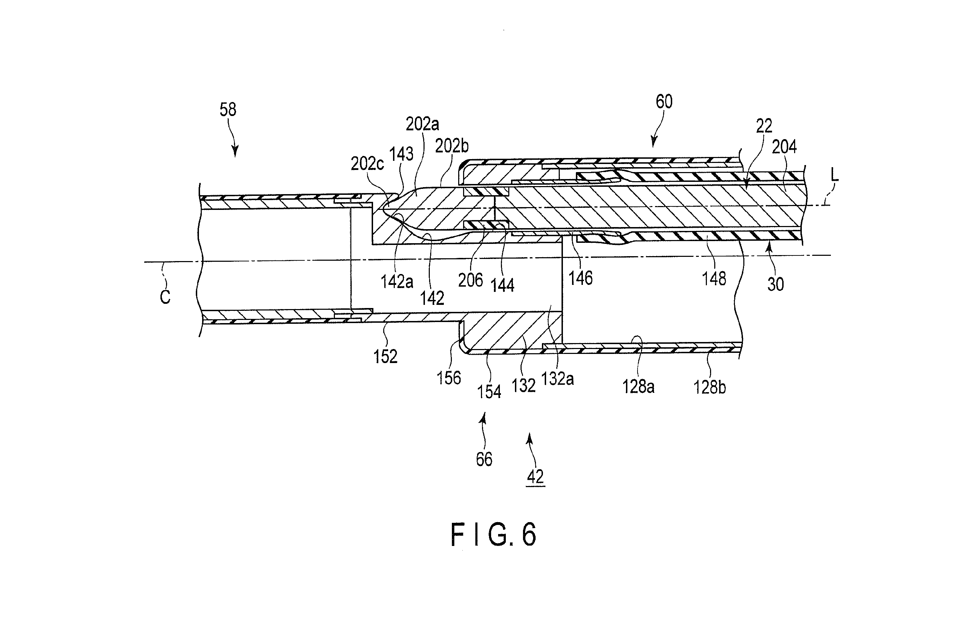

The collar 206 permits the rotation around the longitudinal axis L. The collar 206 may be disposed across the outer circumferential surfaces of the rotation gear 202 and the drive shaft 204 as shown in FIG. 6, or may be provided outside the rotation gear 202 or outside the drive shaft 204. That is, the collar 206 is provided on the outer circumferential surface of at least one of the rotation gear 202 and the drive shaft 204.

As shown in FIG. 9A, the driving source 24 includes a motor main body 212, the rotation shaft 214, and a motor cable 216. The distal end of the motor cable 216 is removably connected to the control unit 14. The outer shape of the cross section of the rotation shaft 214 at right angles with the axial direction of the rotation shaft 214 in the motor main body 212, and outer shape of the cross section of the motor main body 212 are, for example, D-shaped.

As shown in FIG. 9A, the operation portion 34 is provided with the attachment portion 222 which defines an exit 222a of the proximal end of the drive shaft 204 of the rotation force transmission unit 22 and to which the later-described driving source 24 for transmitting rotation force to the proximal end of the drive shaft 204 is attached. A holding ring 224 which holds the outer circumference of the motor main body 212 is provided in the attachment portion 222.

The channel tube 148 shown in FIG. 5 to FIG. 7 is in communication with the exit 222a of the attachment portion 222 through the insertion body 42 and the operation portion 34. Therefore, the proximal end of the channel tube 148 is open at the exit 222a of the attachment portion 222.

In this way, the gear location cavity 142, the support portion 144, the channel mouth ring 146, the channel tube 148, and the attachment portion 222 are defined from the distal end to the proximal end in order in the flexible tube connection portion 66 and the second flexible tube 60 of the insertion body 42, so that the channel 30 as an insertion path through which the rotation force transmission unit 22 is inserted is formed.

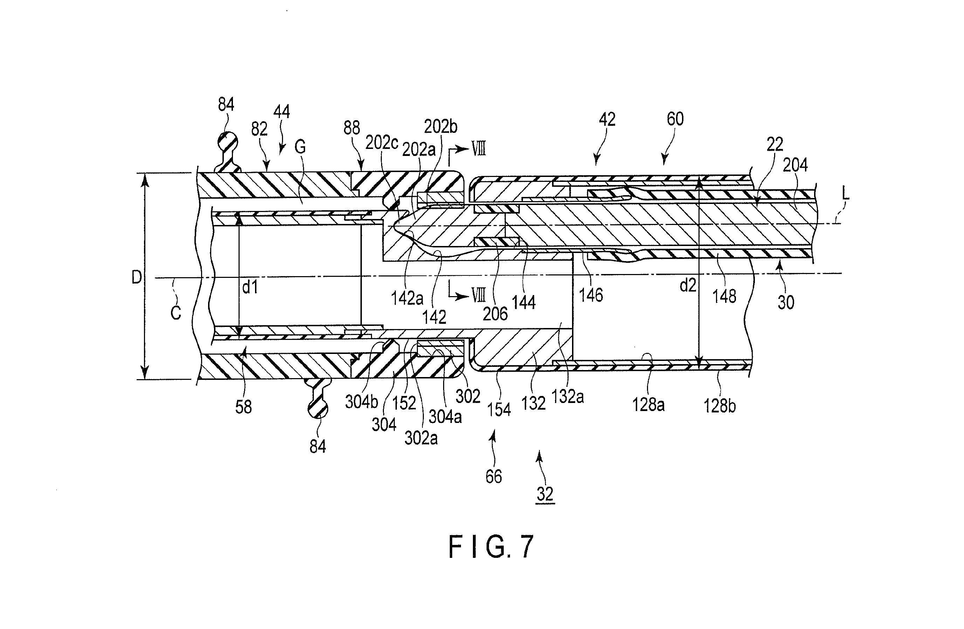

The rotation unit 44 shown in FIG. 7 and FIG. 8 can be attached to and detached from the insertion body 42 through the distal hard portion 52.

The tube body 82 of the rotation unit 44 is made of a resin material such as polyurethane. The tube body 82 has a gap G between the tube body 82 and the outer circumferential surface of the outer tube 108 of the bending portion shown in FIG. 4 as well as the outer circumferential surface of the first flexible tube 58 shown in FIG. 7. That is, the tube body 82 is provided to have the gap G between the tube body 82 and the outer circumferential portion of the insertion body 42. This prevents friction from being caused between the insertion body 42 and the tube body 82 when the rotation unit 44 rotates relative to the insertion body 42.

The tube distal portion 86 is made of a material such as a rubber material softer than the tube body 82. As shown in FIG. 4, the inner circumferential portion of the tube distal portion 86 is formed so that the gap G between the rotation unit 44 and the outer tube 108 of the bending portion is smaller than in the part located on the inner circumferential side of the tube body 82.

According to this embodiment, the tube proximal portion 88 of the rotation unit 44 includes an inner cylindrical body (internal gear) 302 having the internal teeth 302a which can mesh with the external teeth 202b of the rotation gear 202 of the rotation force transmission unit 22, and an outer cylindrical member (tube) 304 which is fixed to the proximal end of the tube body 82 and which is provided outside the inner cylindrical body 302 and which has a cylindrical support portion 304a to support the inner cylindrical body 302 on the inner circumferential surface of the support portion 304a. If the internal teeth 302a of the inner cylindrical body 302 mesh with the external teeth 202b of the rotation gear 202 of the rotation force transmission unit 22, the inner cylindrical body 302 can rotate outside the insertion body 42 around the central axis C. Since the outer cylindrical member 304 supports the inner cylindrical body 302 on its inner circumferential surface, the outer cylindrical member 304 rotates together with the inner cylindrical body 302 if the inner cylindrical body 302 rotates around the central axis C of the insertion body 42.

The inner cylindrical body 302 is preferably made of a metallic material such as stainless steel. The outer cylindrical member 304 is preferably made of an electrically insulating resin or rubber material. The inner cylindrical body 302 and the outer cylindrical member 304 are preferably integrated by, for example, insert molding.

Here, the axial length of the inner cylindrical body 302 along the central axis C is shorter than the axial length of the outer cylindrical member 304 along the central axis C. The entire outer circumferential surface of the inner cylindrical body 302 is covered with the inner circumferential surface (support portion 304a) of the outer cylindrical member 304. The proximal end of the inner cylindrical body 302 and the proximal end of the outer cylindrical member 304 may be located at the same position in the longitudinal direction of the central axis C.

A ring-shaped or properly separated projection (holding portion) 304b which rotatably contacts with the outer circumferential surface of the small-diameter portion 152 of the connection mouth ring 132 is formed in the inner circumferential surface of the outer cylindrical member 304. The projection 304b is preferably integrated with the outer cylindrical member 304 closer to the distal side than the support portion 304a. That is, the outer cylindrical member 304 preferably has the projection 304b.

The projection 304b functions as a posture maintaining portion which maintains the posture of the rotation unit 44 relative to the insertion body 42 so that the central axis C of the insertion body 42 corresponds to the central axis C of the rotation unit 44. The projection 304b also maintains a parallel state of the central axis C of the insertion body 42 and the longitudinal axis L of the gear portion 202b of the rotation gear 202 and maintains a diametrical meshing posture of the external teeth 202b of the rotation gear 202 and the internal teeth 302a of the internal gear 302 while the rotation gear 202 is provided in the opening end portion 143 of the channel 30. Therefore, a given distance between the internal teeth 302a of the inner cylindrical body 302 of the tube proximal portion 88 and the external teeth 202b of the rotation gear 202 is maintained by the projection 304b, and a predetermined meshing state between the internal teeth 302a of the inner cylindrical body 302 and the external teeth 202b of the rotation gear 202 can be maintained. That is, the projection 304b can keep the internal teeth 302a of the inner cylindrical body 302 in mesh with the external teeth 202b of the rotation gear 202 while the rotation gear 202 is provided in the opening end portion 143 of the channel 30.

When the projection 304b is formed as an O-ring, the projection 304b becomes thinner from its outer circumferential surface to its inner circumferential surface, that is, becomes thinner toward the small-diameter portion 152, and it is preferable that its sectional surface is, for example, substantially V-shaped. This shape permits the rotation relative to the insertion body 42 around the central axis C, but prevents the movement of, for example, the distal side of the insertion body 42 in particular in the axial direction of the central axis C owing to friction. That is, the shape of the projection 304b is properly formed so that frictional force is generated between the small-diameter portion 152 of the connection mouth ring 132 and the projection 304b of the outer cylindrical member 304 of the rotation unit 44. The force (force against the frictional force) necessary to move the projection 304b of the rotation unit 44 relative to the connection mouth ring 132 in the axial direction of the central axis C is then compared with the force (force against the frictional force) necessary to rotate the projection 304b relative to the connection mouth ring 132 around the central axis C. In this case, the former force can be sufficiently higher than the latter force.

As shown in FIG. 7, according to this embodiment, an outside diameter D of the outer cylindrical member 304 of the rotation unit 44 is not only larger than the outside diameter d1 of the first flexible tube 58 but also larger than the large-diameter portion 154 of the connection mouth ring 132 and the outside diameter d2 of the second flexible tube 60. However, the outside diameter D of the outer cylindrical member 304 is preferably formed to such a degree that, when compared, the outside diameter D is not different from the outside diameter of the large-diameter portion 154 of the connection mouth ring 132 and the outside diameter d2 of the second flexible tube 60. That is, it is preferable that the difference between the outside diameter D of the outer cylindrical member 304 of the rotation unit 44 and the outside diameter d2 of the second flexible tube 60 is small and that a small step is formed at the boundary. In this way, it is possible to maximally prevent the proximal end of the rotation unit 44 from being caught by, for example, the inner wall of the lumen when the insertion portion 32 is retreated and removed from the lumen.

The minimum inside diameter of the part of the outer cylindrical member 304 other than the projection 304b is larger than the outside diameter of the proximal end of the first flexible tube 58, and the gap G is provided between the inner circumferential surface of an inner cylindrical portion and the outer circumferential surface of the first flexible tube 58.

The fin portion 84 extending on the outer circumferential portion of the tube body 82 is made of, for example, a rubber material. The fin portion 84 is fixed to the tube body 82 by, for example, adhesive bonding or welding. As shown in FIG. 1, the fin portion 84 spirally extends clockwise when viewed from the proximal direction. When the insertion portion 32 of the endoscope 12 is inserted in the lumen in the small intestine or the large intestine, the fin portion 84 of the rotation unit 44 contacts with the wall of the lumen. In this condition, the rotation unit 44 is rotated relative to the insertion body 42 around the central axis C. As a result, propulsion in a direction along the axial direction of the central axis C is applied to the insertion portion 32.

Now, the operation of the endoscope system 10 according to this embodiment is described. Here, an assembly procedure for attaching the rotation force transmission unit 22 and the rotation unit 44 to the insertion body 42 and attaching the driving source 24 to the operation portion 34 is mainly described.

The rotation gear 202 of the rotation force transmission unit 22 shown in FIG. 2A is introduced, from its distal end, into the gear location cavity 142 of the flexible tube connection portion 66 through the attachment portion 222, the channel tube 148, the channel mouth ring 146, and the support portion 144. The rotation gear 202 is disposed in the gear location cavity 142 of the channel 30. That is, the rotation gear 202 is disposed in the opening end portion 143 of the channel 30. At the same time, the longitudinal axis L of the rotation force transmission unit 22 is parallel with the central axis C of the insertion body 42.

In this case, if the whole rotation force transmission unit 22 is pulled toward the rear end of the channel 30, the rotation force transmission unit 22 is easily pulled from the channel 30.

When the rotation force transmission unit 22 is disposed at a predetermined position in the channel 30 as shown in FIG. 6 to FIG. 8, the proximal end of the drive shaft 204 is located in the attachment portion 222 or in the vicinity of the attachment portion 222 as shown in FIG. 9A. In this condition, the driving source 24 shown in FIG. 9A is attached to the D-shaped attachment portion 222 shown in FIG. 9B, and the rotation shaft 214 is fitted into the D-shaped depression 208 at the proximal end of the drive shaft 204.

The rotation unit 44 is then moved toward the proximal end of the insertion body 42 while being rotated around the central axis C. That is, the proximal end of the tube proximal portion 88 of the rotation unit 44 is moved toward the proximal side through the outside of the small-diameter portion 152 of the connection mouth ring 132 of the flexible tube connection portion 66. The internal teeth 302a of the inner cylindrical body 302 then mesh with the gear portion 202b of the rotation force transmission unit 22, and the projection 304b of the outer cylindrical member 304 is brought into rotatable abutment with the outer circumferential surface of the small-diameter portion 152 of the connection mouth ring 132. In this case, there is almost no gap between the proximal end of the tube proximal portion 88 and the step portion 156 at the distal end of the large-diameter portion 154 of the connection mouth ring 132.

Here, the projection 304b of the outer cylindrical member 304 is thinner and in closer contact with the outer circumferential surface on the side closer to the small-diameter portion 152 of the connection mouth ring 132. This permits the rotation relative to the insertion body 42 around the central axis C, but prevents the movement of, for example, the distal side of the insertion body 42 in particular in the axial direction of the central axis C owing to friction.

Thus, the cylindrical rotation unit (insertion assist device) 44 is provided outside the elongated insertion body 42 of the endoscope (insertion apparatus) 12 which defines the central axis C substantially parallel with the longitudinal axis L of the channel 30 having the opening end portion 143 open to the outside and which is able to attach and detach the rotation gear 202 having the external teeth 202b of the rotation force transmission unit 22.

The outside of the small-diameter portion 152 of the connection mouth ring 132 is covered with the rotation unit 44 when the rotation unit 44 is attached to the connection mouth ring 132. Moreover, (at least part of) the proximal end of the first flexible tube 58 coupled to the distal side of the small-diameter portion 152 is covered with the rotation unit 44.

The insertion portion 32 of the endoscope 12 is available in this state. That is, the rotation unit 44 of the insertion portion 32 is rotatable relative to the central axis C in the first direction and the second direction.

For example, when the rotation unit 44 of the insertion portion 32 is rotated in the first direction, the position indicated by the sign 78a in the rotational operation input switch 78 shown in FIG. 1 and FIG. 3 is pressed while the endoscope system 10 shown in FIG. 1 is active. A signal of the pressing of the rotational operation input switch 78 is input to the control unit 14 via the universal cable 36 and the connector 36a. The control unit 14 rotates the rotation shaft 214 of the driving source 24 in the first direction via the motor cable 216 and the motor main body 212. In response to the rotation of the rotation shaft 214 in the first direction, the drive shaft 204 rotates in the first direction. As a result of the rotation of the drive shaft 204 in the first direction, the rotation force is transmitted to the rotation gear 202, and the rotation gear 202 rotates in the first direction. Therefore, the inner cylindrical body 302 having the internal teeth 302a in mesh with the rotation gear 202 rotates in the first direction. Together with the rotation of the inner cylindrical body 302 in the first direction, the outer cylindrical member 304 integrated with the inner cylindrical body 302 rotates in the first direction.

When the rotation unit 44 of the insertion portion 32 is rotated in the second direction, the position indicated by the sign 78b in the rotational operation input switch 78 is pressed while the endoscope system 10 shown in FIG. 1 is active.

According to the present embodiment, the fin portion 84 spirally extends clockwise when viewed from the proximal direction. Therefore, if the rotation unit 44 rotates clockwise (in the first direction) when viewed from the proximal direction, propulsive force in the distal direction is applied to the insertion portion 32. Thus, the insertability of the insertion portion 32 in the lumen is improved. On the other hand, if the rotation unit 44 rotates counterclockwise (in the second direction) when viewed from the proximal direction, propulsive force in the proximal direction is applied to the insertion portion 32. Thus, the removability of the insertion portion 32 in the lumen is improved.

The difference (step) between the outside diameter D of the outer cylindrical member 304 of the rotation unit 44 and the outside diameter d2 of the second flexible tube 60 according to this embodiment is small. Therefore, for example, when the insertion portion 32 is removed from the lumen, it is possible to maximally prevent a living tissue from being caught between the proximal end of the outer cylindrical member 304 of the rotation unit 44 and the step portion 156 of the connection mouth ring 132. Since the gap between the proximal end of the outer cylindrical member 304 of the rotation unit 44 and the step portion 156 of the connection mouth ring 132 is small, it is possible to maximally prevent the living tissue from being caught between the proximal end of the outer cylindrical member 304 of the rotation unit 44 and the step portion 156 of the connection mouth ring 132, for example, when the insertion portion 32 is removed from the lumen.

After the use of the endoscope 12, the endoscope 12 is washed, disinfected, and sterilized, and then reused. This procedure is briefly described.

The rotation unit 44 is gradually pulled toward the distal side against the frictional force while being rotated around the central axis C of the insertion body 42. Therefore, the rotation unit 44 is moved from the outside of the flexible tube connection portion 66 to the distal side. The meshing of the gear portion 202b of the rotation gear 202 with the internal teeth 302a of the inner cylindrical body 302 of the rotation unit 44 is released. The rotation unit 44 is then detached from the distal end of the insertion body 42.

The driving source 24 and the rotation force transmission unit 22 are then pulled from the proximal end of the channel 30 toward the near side. Therefore, the rotation force transmission unit 22 can be removed from the channel 30. The rotation force transmission unit 22 is then completely removed from the channel 30 as shown in FIG. 5. Here, the rotation force transmission unit 22 can be easily attached to and detached from the channel 30.

The rotation force transmission unit 22 may be detached from the insertion body 42 after the rotation unit 44 is detached from the insertion body 42. The rotation unit 44 may be detached from the insertion body 42 after the rotation force transmission unit 22 is detached from the insertion body 42.

The insertion path can be then ensured when, for example, a washing fluid or a brush is inserted into the channel 30 through the attachment portion 222, the channel tube 148, the channel mouth ring 146, the support portion 144, and the gear location cavity 142. This ensures that the inside of the channel 30 can be washed by the use of an unshown brush or the like while the rotation unit 44 and the rotation force transmission unit 22 are removed from the insertion portion 32. Therefore, the insertion body 42 and the operation portion 34 can be easily washed, disinfected, and sterilized.

As described above, the following can be said according to this embodiment.

The rotation unit 44 with which the inner cylindrical body 302 having the internal teeth 302a is integrated can be easily attached to and detached from the insertion body 42. Thus, the insertion body 42 has no internal teeth to mesh with the external teeth 202b of the rotation gear 202 of the rotation force transmission unit 22 when the channel 30, that is, the insertion path to insert the rotation force transmission unit 22 is washed. Consequently, the washing performance of the channel 30 can be improved.

The projection 304b is formed in the outer cylindrical member 304 of the tube proximal portion 88 of the rotation unit 44 at the position adjacent to the inner cylindrical body 302. This projection 304b can keep the longitudinal axis (rotation axis) L of the rotation gear 202 of the rotation force transmission unit 22 parallel with the central axis C of the internal teeth 302a of the rotation unit 44. This ensures that the rotation of the rotation gear 202 of the rotation force transmission unit 22 around the longitudinal axis L can be transmitted to the rotation unit 44 through the internal teeth 302a.

When the rotation unit 44 is attached to the insertion body 42, no additional components for the attachment are needed. That is, when the rotation unit 44 is attached to the insertion body 42, the rotation unit 44 has only to be moved to the proximal side along the central axis C relative to the insertion body 42 while being rotated so that the projection 304b of the outer cylindrical member 304 is in abutment with the outer circumferential surface of the small-diameter portion 152 of the connection mouth ring 132 in particular. Thus, the rotation unit 44 can be easily attached to the insertion body 42.

When the rotation unit 44 is detached from the insertion body 42, no additional components for the detachment are needed. That is, when the rotation unit 44 is detached from the insertion body 42, the rotation unit 44 has only to be moved to the distal side along the central axis C relative to the insertion body 42 while being rotated so that the projection 304b of the outer cylindrical member 304 is in contact with the outer circumferential surface of the small-diameter portion 152 of the connection mouth ring 132 in particular. Thus, the rotation unit 44 can be easily detached from the insertion body 42.

Consequently, in the endoscope 12 according to this embodiment, the rotation unit 44 can be easily attached to and detached from the insertion body 42, and assembling efficiency can be improved.

When the rotation force transmission unit 22 is attached to the insertion body 42, the rotation gear 202 has only to be moved toward the gear location cavity 142 relative to the channel 30. When the rotation force transmission unit 22 is detached from the insertion body 42, the rotation gear 202 has only to be moved away from the gear location cavity 142 relative to the channel 30.

Consequently, in the endoscope 12 according to this embodiment, the rotation force transmission unit 22 can be easily attached to and detached from the insertion body 42, and assembling efficiency can be improved.

Thus, when the endoscope 12 is washed after use, the rotation force transmission unit 22 and the rotation unit 44 can be easily detached, so that the washing performance can be improved.

Therefore, according to this embodiment, it is possible to provide the rotation unit (insertion assist device) 44 which can be easily attached to and detached from the insertion body 42 of the insertion portion 32 to improve the washing performance of the insertion body 42, the insertion body 42 which can improve the washing performance while the rotation unit 44 is detached, and the endoscope (insertion apparatus) 12 having the insertion body 42 and the rotation unit 44.

The rotation force transmission unit 22 according to this embodiment is supported by what is known as double supported holding to rotate so that the projection 202c at the distal end of the rotation gear 202 is supported by the support depression 142a of the channel 30 and so that the collar 206 at the proximal end of the rotation gear 202 is supported by the support portion 144 of the channel 30. Thus, it is possible to effectively prevent the rotation gear 202 from being out of alignment with the longitudinal axis L while being rotated.

Although a motor is used as the driving source 24 in the example described according to this embodiment, the rotation shaft 214 may be manually rotated.

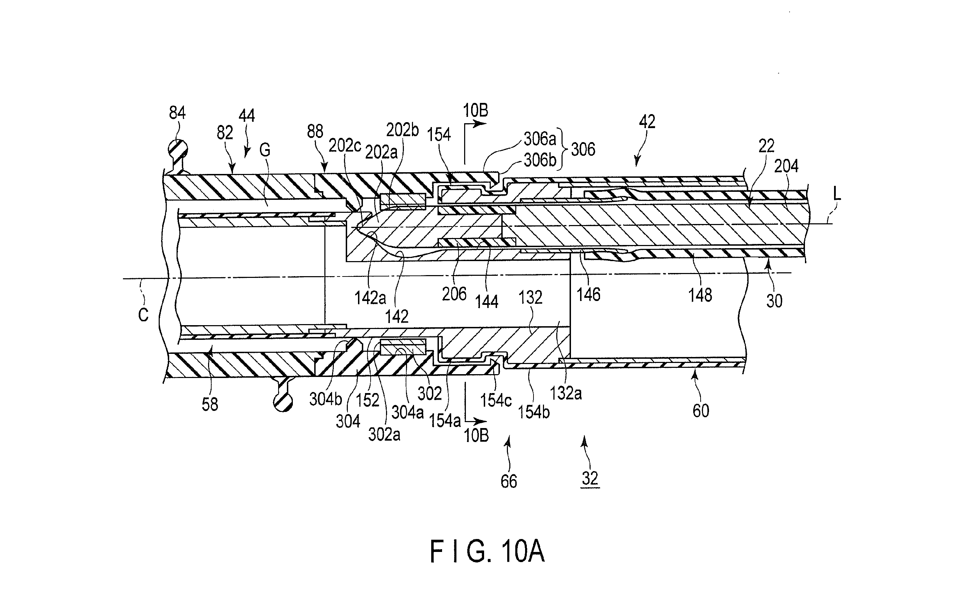

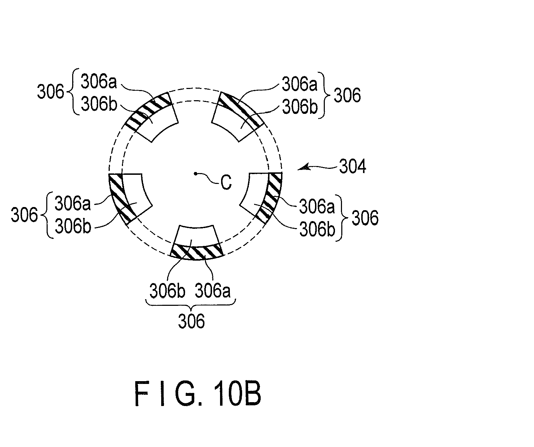

Now, the second embodiment is described with reference to FIG. 10A and FIG. 10B. This embodiment is a modification of the first embodiment, and the same components as the components described in the first embodiment are indicated by the same reference signs and are not described in detail.

In the example described according to this embodiment, the shape of the connection mouth ring 132 of the flexible tube connection portion 66 of the insertion body 42, and the shape of the outer cylindrical member (tube) 304 of the tube proximal portion 88 of the rotation unit 44 are changed from those in the first embodiment.

According to this embodiment, the support portion 304a of the outer cylindrical member (tube) 304 of the tube proximal portion 88 of the rotation unit 44 holds not only the distal side of the inner cylindrical body 302 but also the proximal side thereof.

As shown in FIG. 10A, the large-diameter portion 154 of the connection mouth ring 132 includes a first-diameter portion 154a larger in diameter than the small-diameter portion 152 and smaller in diameter than the maximum diameter of the large-diameter portion 154, a second-diameter portion 154b which forms the maximum diameter of the large-diameter portion 154, and an annular groove 154c formed between the first-diameter portion 154a and the second-diameter portion 154b. The first-diameter portion 154a and the second-diameter portion 154b are concentric with the central axis C. The first-diameter portion 154a is located adjacent to the proximal side of the small-diameter portion 152. The second-diameter portion 154b is located on the proximal side of the first-diameter portion 154a, and located apart from the proximal side of the small-diameter portion 152.

As shown in FIG. 10A and FIG. 10B, holding pieces (holding portions) 306 which can be fitted into the annular groove 154c and which are elastically deformable in the diametric direction relative to the central axis C are formed on the proximal side of the outer cylindrical member 304. That is, the inner cylindrical body (internal gear) 302 is provided between the projection 304b of the outer cylindrical member 304 and the holding pieces 306. As shown in FIG. 10B, the holding pieces 306 are provided at proper intervals. The holding pieces 306 are preferably integrated with the outer cylindrical member 304. That is, the outer cylindrical member 304 preferably has the holding pieces 306.

Each of the holding pieces 306 includes an extension (depression) 306a which covers the outer circumference from the distal end of the first-diameter portion 154a to the proximal side, and a claw (projection) 306b which is formed at the proximal end of the extension 306a and which diametrically inwardly projects to be engageable with the annular groove 154c. The amount of the diametrically inward projection of the claw 306b (the depth of the annular groove 154c) has only to be such that the rotation unit 44 can be kept held to the insertion body 42 in the axial direction of the central axis C. It is preferable that the inner circumferential surfaces of the claws 306b are formed so that this inner circumferential surface does not contact the external teeth 202b of the rotation gear 202 when the rotation unit 44 is attached to and detached from the insertion body 42 while the rotation gear 202 is disposed in the gear location cavity 142.

Thus, the claws 306b of the outer cylindrical member 304 engage with the annular groove 154c of the large-diameter portion 154 of the connection mouth ring 132, so that the claws 306b are used as a displacement prevention portion which prevents the displacement of the insertion body 42 in the axial direction of the central axis C. In this case, when the claws 306b of the outer cylindrical member 304 engage with the annular groove 154c of the connection mouth ring 132, the internal teeth 302a of the inner cylindrical body 302 can be kept in mesh with the gear portion 202b of the rotation gear 202 while the rotation gear 202 is disposed in the opening end portion 143 of the channel 30 because the movement of the rotation unit 44 in the axial direction of the central axis C of the insertion body 42 is prevented.

The claws 306b are formed into inclined surfaces so that the holding pieces 306 are elastically deformed diametrically outward when the proximal-side inner circumferential surfaces of the distal ends (diametrically inward parts) relative to the claws 306b of the holding pieces 306 have come into abutment with the distal end of the first-diameter portion 154a of the large-diameter portion 154. The claw 306b is formed to maintain the engaging state so that the distal side of its distal end relative to the extension 306a is in engagement with the annular groove 154c. Specifically, the distal side of the distal end of the claw 306b relative to the extension 306a is shaped to form, for example, a surface that is inclined to the proximal side outside a surface intersecting at right angles with the central axis C.

The part between the wall surface of the annular groove 154c and each of the claws 306b is made of a material which minimizes friction, and is formed so as to minimize friction.

Now, the operation of the endoscope system 10 according to this embodiment is described. Here, a procedure for attaching the rotation unit 44 to the insertion body 42 and a procedure for detaching are briefly described.

First, the procedure for attaching the rotation unit 44 to the insertion body 42 is described.

The rotation unit 44 is moved to the proximal side of the insertion body 42 while being rotated around the central axis C. That is, the proximal end of the tube proximal portion 88 of the rotation unit 44 is moved to the proximal side through the outside of the small-diameter portion 152 of the connection mouth ring 132 of the flexible tube connection portion 66.

If the proximal-side inner circumferential surfaces of the distal ends (diametrically inward parts) of the claws 306b relative to the extensions 306a of the holding pieces 306 have come into contact with the distal end of the first-diameter portion 154a of the large-diameter portion 154, the extensions 306a of the holding pieces 306 are elastically deformed diametrically outward.

In this condition, the rotation unit 44 is further moved to the proximal side of the insertion body 42 while being rotated around the central axis C. Thus, the claws 306b of the holding pieces 306 come into the annular groove 154c, and the extensions 306a are restored to the original state.

Since the claws 306b are in the annular groove 154c, the claws 306b and the annular groove 154c prevent displacement that causes the rotation unit 44 to move in the axial direction of the central axis C of the insertion body 42.

The projection 304b of the outer cylindrical member 304 is thinner and in closer contact with the outer circumferential surface on the side closer to the small-diameter portion 152 of the connection mouth ring 132. This permits the rotation relative to the insertion body 42 around the central axis C. The projection 304b maintains a given distance between the small-diameter portion 152 of the connection mouth ring 132 and the inner circumferential surface of the outer cylindrical member 304. Thus, the posture of the rotation unit 44 relative to the connection mouth ring 132 of the insertion body 42 can be maintained by the projection 304b.

Now, the procedure for detaching the rotation unit 44 from the insertion body 42 is described.

The extensions 306a and the claws 306b of the holding pieces 306 are opened to the outside, and the claws 306b are then put out of the annular groove 154c. The rotation unit 44 is then gradually pulled toward the distal side against the frictional force while being rotated around the central axis C of the insertion body 42. In this way, the rotation unit 44 is moved to the distal side from the outside of the flexible tube connection portion 66. The rotation unit 44 is then detached from the distal end of the insertion body 42.

When the rotation unit 44 is disposable, the extensions 306a may be folded to take the claws 306b out of the annular groove 154c, or the extensions 306a may be cut to disengage the holding pieces 306 and the annular groove 154c.

In the example described according to this embodiment, the claws 306b of the rotation unit 44 engage with the annular groove 154c of the large-diameter portion 154 of the connection mouth ring 132. The outer circumferential surface of the first-diameter portion 154a of the large-diameter portion 154 of the connection mouth ring 132 may be formed as a projection, and the space formed by the proximal end of the inner cylindrical body (internal gear) 302, by the extensions 306a, and by the claws 306b may be a depression, which enables the projection and the depression to engage with each other.

Although the rotation unit 44 is attached to the insertion body 42 of the endoscope 12 in the examples described above according to the first and second embodiments, this invention is not limited to the endoscope 12. For example, it is possible to use a structure that permits the rotation unit 44 to be attached to and detached from the insertion body 42 of, for example, a surgical manipulator (insertion apparatus).

Although the several embodiments have been specifically explained with reference to the drawings, the present invention is not restricted to the foregoing embodiments, and it includes all embodiments effected without departing from the gist thereof.

### Reference Signs List

C···central axis, L···longitudinal axis (rotation axis), 12···endoscope, 22···rotation force transmission unit, 24··· driving source, 30···channel, 32···insertion portion, 42··· insertion body, 44···rotation unit, 58···first flexible tube, 60···second flexible tube, 66···flexible tube connection portion, 82···tube body, 84···fin portion, 88···tube proximal portion, 128a···tubular member, 128b···outer tube, 132··· connection mouth ring, 132a···cavity, 142···gear location cavity, 142a···support depression, 143···opening end portion, 144···support portion, 146···channel mouth ring, 148···channel tube, 152···small-diameter portion, 154···large-diameter portion, 154a···first-diameter portion, 154b···wsecond-diameter portion, 154c···annular groove, 156···step portion, 202···rotation gear, 202a···main body, 202b···rotation gear portion (external teeth), 202c···projection, 204···drive shaft, 206···collar, 302···inner cylindrical body (internal gear), 302a···internal teeth, 304···outer cylindrical member (tube), 304a···support portion, 304b···projection, 306··· holding pieces, 306a···extension, 306b···claw.

## Claims

1. A cylindrical insertion assist device in an insertion apparatus, the insertion assist device provided outside an elongated insertion body which defines a central axis being substantially parallel with a longitudinal axis of a channel having an opening end portion in which a rotation gear having external teeth of a rotation force transmission unit is detachably provided and which is open to the outside, the insertion assist device comprising:
an internal gear which includes internal teeth configured to mesh with the external teeth of the rotation gear and which is rotatable around the central axis while the rotation gear is disposed in the opening end portion of the channel; and
a tube which is configured to support the internal gear on an inner circumferential surface of the tube.

2. The insertion assist device according to claim 1, wherein the tube includes a holding portion which is configured to hold a state that the internal teeth of the internal gear is in mesh with the external teeth of the rotation gear while the rotation gear is disposed in the opening end portion of the channel.

3. The insertion assist device according to claim 2, wherein the holding portion includes a displacement prevention portion which prevents the displacement of the insertion body in the axial direction of the central axis.

4. The insertion assist device according to claim 2, wherein the holding portion includes a posture maintaining portion which is configured to maintain a parallel state of the central axis of the insertion body and a rotation axis of the external teeth of the rotation gear and which is configured to maintain a meshing posture in a radial direction between the external teeth of the rotation gear and the internal teeth of the internal gear while the rotation gear is provided in the opening end portion of the channel.

5. The insertion assist device according to claim 2,
wherein
at least two holding portions are provided, and
the internal gear is provided between the at least two holding portions.

6. An insertion body in an insertion apparatus, the insertion body comprising:
a cylindrical base, the insertion assist device according to claim 1 being detachably provided outside the base; and
a channel provided inside the base, the channel having an opening end portion which is open to the outside of the base so that external teeth provided on a rotation gear of a rotation force transmission unit are configured to be disposed in mesh with the internal gear of the insertion assist device.

7. The insertion body according to claim 6, wherein the base includes:
a small-diameter portion coupled to a first flexible tube which is at least partly covered with the insertion assist device while the insertion assist device is attached to the base, and
a large-diameter portion which is larger in diameter than the small-diameter portion, which is concentric with the small-diameter portion and which is located on the proximal side of the small-diameter portion, the large-diameter portion being coupled to a second flexible tube different from the first flexible tube.

8. The insertion body according to claim 6, wherein at least part of the external teeth of the rotation gear is exposed to the outside of the base in the opening end portion of the channel while the rotation gear is provided in the opening end portion of the channel.

9. An insertion apparatus comprising:
an insertion body according to claim 6;
an insertion assist device according to claim 1 which is detachably provided outside the base of the insertion body; and
a rotation force transmission unit which is provided detachably from a channel of the insertion body and which includes a rotation gear having the external teeth,
wherein:
the external teeth of the rotation gear of the rotation force transmission unit mesh with the internal teeth of the internal gear of the insertion assist device while the rotation force transmission unit is provided in the channel and while the insertion assist device is provided outside the insertion body, and
the outside diameter of the tube of the insertion assist device is larger than the outside diameter of the proximal side of the insertion body at a position where the insertion assist device is provided while the insertion assist device is provided outside the insertion body.
